# EUROPEAN PATENT APPLICATION

(11) **EP 3 375 732 A1**
(43) Date of publication of application: **19.09.2018**
(21) Application number: 16864034.0
(22) Date of filing: 27.10.2016
(51) Int. Cl.: B65D 83/68, A61K 8/22, A61K 8/36, A61K 8/39, A61K 8/86, A61Q 5/08, A61Q 5/10

(54) **AEROSOL CONTAINER HAVING DOUBLE STRUCTURE**

(30) Priority: 10.11.2015 JP 2015220390
(71) Applicant: Toyo Seikan Co., Ltd., Tokyo 141-8640 (JP)
(72) Inventor: YAMAGUCHI, Yuuji, Yokohama-shi, Kanagawa 230-0001 (JP); TANI, Kiyohiro, Yokohama-shi, Kanagawa 230-0001 (JP)
(74) Representative: J A Kemp
(86) International application number: PCT/JP2016/081904
(87) International publication number: WO 2017/082070

(57) **Abstract**

The present invention relates to a double-structure aerosol container for a two-agent type hair cosmetic, having an outer vessel and an inner bag to be housed in the outer vessel. An inner bag includes an inner layer and an outer layer both of which are formed of a polyolefin resin and an intermediate layer formed of a cyclic olefin copolymer or an amorphous polyester resin, thereby preventing an inner surface of the outer vessel from corrosion after storing at high temperature with the inner bag filled with a second agent containing hydrogen peroxide as a major ingredient and at least one of an organic acid or an organic acid compound.

## Description

### Technical Field:

The present invention relates to a double-structure aerosol container for a two-agent type hair cosmetic. More specifically, the present invention relates to a double-structure aerosol container for a two-agent type hair cosmetic, including an outer vessel and an inner bag with an excellent barrier property to an organic acid derived from a specific component contained in the two-agent type hair cosmetic, thereby preventing effectively corrosion of the inner surface of the outer vessel.

### Background Art:

A two-agent type hair cosmetic such as a two-agent type hair dye or a two-agent type hair bleaching agent is composed of a first agent containing an alkaline component and a second agent containing hydrogen peroxide. The component of the first agent is chemically deteriorated when it comes into contact with oxygen during storage to cause decline in the hair dyeing function.

The second agent includes an oxidizing acidic component containing hydrogen peroxide as a major ingredient. Therefore, the inner bag to contain the second agent is required to have resistance to an internal expansion pressure caused by oxygen generated due to decomposition of hydrogen peroxide which serves as an oxidant. Other problems to be solved include loss of effective components, which may occur when the hydrogen peroxide in the content permeates the inner bag. Furthermore, when the outer vessel is a metallic container, it is necessary to prevent the inner surface of the outer vessel from corrosion caused by the component permeated through the inner bag.

As a container for packaging such a two-agent type hair cosmetic having the aforementioned features, a double-structure aerosol container has been provided. The double-structure aerosol container includes inner bags each of which contains a first agent and a second agent and an outer vessel in which the inner bags are housed. For instance, Patent document 1 below describes a duplex aerosol container formed by connecting two double-structure aerosol containers. The double-structure aerosol containers are prepared by housing an inner bag filled with a first agent and another inner bag filled with a second agent in an outer bag. This duplex aerosol container is capable of simultaneously ejecting the first agent and the second agent. In this Patent document 1, as an inner cylinder to be housed in the outer vessel, an inner cylinder made from a synthetic resin obtained by mixing a polymeric antioxidant with a linear low-density polyethylene has been proposed.

### Prior Art Documents:

### Patent Documents:

Patent Document 1: JP-A-2005-15017

### Outline of the Invention:

### Problems that the Invention is to Solve:

As mentioned above, the double-structure aerosol container of Patent document 1 includes an inner bag of a linear low-density polyethylene, which is filled with the second agent of the two-agent type hair cosmetic. The inner bag is then housed in a metallic outer vessel. However, the double-structure aerosol container has been found to involve a problem that corrosion may occur on the inner surface of its outer vessel over time when the second agent contains a certain component.

Therefore, the purpose of the present invention is to provide a double-structure aerosol container including an outer vessel with an inner surface free from corrosion, even when the inner bag is filled with a second agent containing a certain component together with hydrogen peroxide for a two-agent type hair cosmetic and then stored under a high-temperature condition.

### Means for Solving the Problems:

The present invention provides a double-structure aerosol container for a two-agent type hair cosmetic, having an outer vessel and an inner bag to be housed in the outer vessel, wherein the inner bag includes an inner layer and an outer layer both of which are formed of a polyolefin resin and an intermediate layer formed of a cyclic olefin copolymer or an amorphous polyester resin, and the two-agent type hair cosmetic includes a second agent that contains hydrogen peroxide as a major ingredient and that contains at least one of an organic acid and an organic acid compound.

It is preferable in the double-structure aerosol container of the present invention that:
1. the amorphous polyester resin is amorphous glycol-modified polyethylene terephthalate;
2. the second agent contains polyoxyethylene alkyl ether;
3. the organic acid and an organic acid in the organic acid compound are at least one of acetic acid, formic acid and oxalic acid;
4. the inner bag has a thickness in a range of 300 to 700 µm;
5. the outer vessel is made from an aluminum seamless can; and
6. a concentration of oxalic acid in a gap between the outer vessel and the inner bag filled with the second agent is not more than 0.5 ppm after storage for six months at 45°C.

### Effects of the Invention:

In the present invention, the inner bag for containing at least the second agent incudes an intermediate layer formed of either a cyclic olefin copolymer or an amorphous polyester resin, and an inner layer and an outer layer both of which are formed of a polyolefin resin. As a result, even in a case of storage for six months under a condition of high temperature of 45°C, permeation of the organic acid can be effectively prevented, and thus, corrosion of the inner surface of the outer vessel can be effectively prevented.

This effect is demonstrated also by the results of Examples described below.

In the Examples, an inner bag filled with an aqueous solution of organic acids comprising acetic acid, formic acid and oxalic acid is prepared, tightly sealed, and stored at 45°C for six months. When the inner bag has a single layer structure of a linear low-density polyethylene (Comparative Examples 1 and 2), a large amount of acetic acid permeates the inner bag. In contrast, when the inner bag has an intermediate layer of a cyclic olefin copolymer or an amorphous polyester resin together with the inner and outer layers of linear low-density polyethylene (Examples 1 and 2), an amount of acetic acid which permeates through the inner bag remarkably decreases in comparison with the inner bag consisting of a single layer of linear low-density polyethylene.

Alternatively, a double-structure aerosol container of the present invention is prepared by filling an inner bag with a second agent containing hydrogen peroxide and polyoxyethylene alkyl ether, tightly sealing the inner bag, and housing the inner bag in an aluminum outer vessel. In a case where the double-structure aerosol container is stored at 45°C for six months, the concentration of oxalic acid which permeates the inner bags is not more than 0.5 ppm (in Example 1, 0.3 ppm and in Example 2, 0.2 ppm), while in Comparative Examples 1 and 2, the concentration of oxalic acid which permeates the inner bags is 1.6 ppm. Apparently in Examples 1 and 2, the permeation amount of oxalic acid remarkably decreases, indicating that corrosion does not occur.

### Brief Description of the Drawings:

[Fig. 1] : a view showing an example of layer constitution of an inner bag for a double-structure aerosol container of the present invention;
[Fig. 2]: a view showing an example of a double-structure aerosol container of the present invention; and
[Fig. 3]: a set of views for indicating a duplex double-structure aerosol container prepared by connecting double-structure aerosol containers as shown in Fig. 2.

### Modes for Carrying Out the Invention:

As described above, as a result of keen studies on corrosion of outer vessels of double-structure aerosol containers, the present inventors have found that the corrosion is caused by organic acids such as acetic acid, formic acid and oxalic acid contained in the second agent, and found that permeation of the organic acids can be prevented by the use of a cyclic olefin copolymer or an amorphous polyester resin for an intermediate layer of the inner bag.

Specifically, the organic acid such as oxalic acid, which is regarded as a corrosive substance, is not limited to the organic acid contained in the second agent from the time of preparation. When a polyoxyethylene alkyl ether-based surfactant is contained in the second agent, the polyoxyethylene alkyl ether may generate ethylene glycol as an intermediate by the active oxygen of the hydrogen peroxide in the reaction illustrated below. Due to this reaction, acetic acid, formic acid and oxalic acid may be generated in the end also from the polyoxyethylene alkyl ether and ethylene glycol. As a result, it is considered that the amounts of organic acids including acetic acid, formic acid and oxalic acid contained in the second agent may be increased in comparison with the time of preparation.

The organic acids in the present invention indicate both the organic acids contained in the second agent from the time of preparation and also the organic acids generated over time.

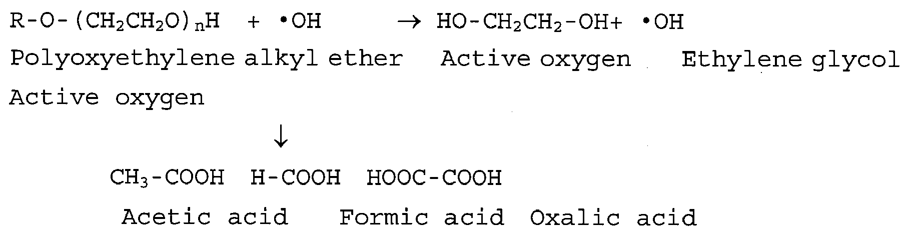

The mechanism of corrosion of the outer vessel, which is caused by the organic acids such as acetic acid, formic acid and oxalic acid generated from the second agent, is assumed as follows.

Specifically, acetic acid, formic acid and oxalic acid generated from the second agent may permeate the inner bag composed of a single layer of linear low-density polyethylene to reach a gap between the inner surface of the outer vessel and the outer surface of the inner bag, and then may adhere to a coating film on the inner surface of the outer vessel. Similarly, hydrogen peroxide passing through the inner bag permeates the coating film on the inner surface of the outer vessel, thereby degrading the adhesion of the coating film. It is therefore considered that the hydrogen peroxide promote the permeation of the organic acids through the coating film, whereby the organic acids reach the metal of the outer vessel to cause corrosion.

It is also clarified by the results in Examples described below that oxalic acid permeates less than acetic acid and formic acid. However, only oxalic acid may form a chelate with aluminum constituting the outer vessel and may remain as aluminum oxalate tetrahydrate at the corrosion site.

### (Inner bag)

An sighnificant feature of the present invention is to use a cyclic olefin copolymer or an amorphous polyester resin for the intermediate layer constituting the inner bag. Moreover, a polyolefin resin is used as the inner and outer layers.

### [Intermediate layer]

An example of the cyclic olefin copolymer (COC) that can be used for the intermediate layer of the present invention is an amorphous or a low crystalline copolymer of an olefin and a cyclic olefin.

For the olefin, ethylene is preferably used, and α-olefins having carbon number of 3 to 20 such as propylene, 1-butene, 1-pentene, 1-hexene, 1-octene, 3-methyl-1-pentene, and 1-decene are also used singly or in combination with ethylene.

A representative and typical example of the cyclic olefin is an aliphatic hydrocarbon compound (e.g., cyclohexene) having an ethylenically unsaturated bond and a bicyclo ring. In addition to that, a bicyclic ring that has a bridging group (e.g. , methylene group and ethylene group) in the cyclohexene ring, and an aliphatic hydrocarbon that has a polycyclic structure having a bycliclic ring and an aliphatic ring further bonded to the bicyclic ring are also preferred. In particular, a hydrocarbon compound having bicyclo [2,2,1] hept-2-ene skeleton is preferred.

An example preferably used as an amorphous polyester resin for the intermediate layer of the present invention is an amorphous polyester resin having an ethylene terephthalate unit and a small amount of copolyester unit.

Examples of copolymerizable components for forming the copolyester include: dicarboxylic acid components such as isophthalic acid, p-β-oxyethoxybenzoic acid, naphthalene 2,6-dicarboxylic acid, diphenoxyethane-4,4'-dicarboxylic acid, 5-sodium sulfoisophthalic acid, adipic acid, sebacic acid or alkyl ester derivatives thereof; and glycol components such as propylene glycol, 1,4-butanediol, neopentyl glycol, 1,6-hexylene glycol, cyclohexanedimethanol, ethylene oxide adduct of bisphenol A, diethylene glycol, and triethylene glycol.

In the present invention, amorphous glycol-modified polyester is particularly preferred. In the glycol-modified polyester, 10 to 50 mol% of the glycol component is glycol for modification. In particular, amorphous polyethylene terephthalate (PETG), in which 10 to 50 mol% of all glycol components are cyclohexanedimethanol (CHDM), can be preferably used.

### [Inner layer and outer layer]

Examples of polyolefin resins constituting the inner and outer layers include low-density polyethylene (LDPE), medium-density polyethylene (MDPE), high-density polyethylene (HDPE), linear low-density polyethylene (LLDPE), linear very low-density polyethylene (LVLDPE), isotactic or syndiotactic polypropylene (PP), ethylene-propylene copolymer, polybutene-1, ethylene-butene-1 copolymer, propylene-butene-1 copolymer, and ethylene-propylene-butene-1 copolymer. Among them, the linear low-density polyethylene can be preferably used, particularly, from the viewpoint of stress crack resistance.

The linear low-density polyethylene that can preferably be used has a melt flow rate (ASTM D1238 at 190°C) in a range of 0.1 to 3 g/10 min., and a density in a range of 0.91 to 0.94 g/cm³.

### [Adhesive layer]

An adhesive layer is preferably provided since aforementioned cyclic olefin copolymer and the amorphous polyester resin have poor adhesion to the polyolefin resin that constitutes the inner and outer layers.

When the intermediate layer is formed of a cyclic olefin copolymer, the preferred embodiments of an adhesive resin constituting the adhesive layer, from the viewpoint of increasing the peel strength between the cyclic olefin and the adhesive resin after a period of time, are described below but are not limited thereto: a main component is (i) an acid-modified product of polyethylene or (ii) an acid-modified product of ethylene-α-olefin copolymer obtained by copolymerizing ethylene with an α-olefin in an amount of 4 to 20 mol%, in particular 10 to 20 mol% based on the resin; and the total density of the adhesive resin is greater than 0.880 g/cm³ and smaller than 0.940 g/cm³, and particularly preferably, not smaller than 0.910 g/cm³.

The polyethylene used preferably has a melt flow rate (ASTM D1238 at 190°C) in a range of 0.4 to 30 g/10 min., and in particular in a range of 1 to 20 g/10 min.

The α-olefin used preferably may be α-olefins having carbon number of 4 to 8 such as butene-1, pentene-1, hexene-1, octene-1, and 4-methylpentene-1. Specific examples of the copolymer include ethylene-butene-1 copolymer, ethylene-pentene-1 copolymer, ethylene-hexene-1 copolymer, ethylene-octene-1 copolymer, and ethylene-4-methylpentene-1 copolymer. These can be used singly, or as a blend of two or more thereof.

Under the condition that the copolymerization ratio of the α-olefin and the density, as a whole lie within the aforementioned ranges, a linear low-density polyethylene can be used as a blend with an ordinary linear middle- or high-density polyethylene.

For the aforementioned acid-modified product of polyethylene or ethylene-α-olefin copolymer, it is particularly preferable to use a graft copolymer prepared by grafting an unsaturated carboxylic acid or an anhydride thereof onto the polyethylene or the ethylene-α-olefin copolymer.

Examples of the unsaturated carboxylic acid and the anhydride thereof include: monocarboxylic acids such as acrylic acid and methacrylic acid; polyvalent carboxylic acids such as maleic acid, fumaric acid, and itaconic acid; polyvalent carboxylic acid anhydrides such as maleic anhydride, itaconic anhydride, and norbornene-2,3-dicarboxylic anhydride; and monoesters of polyvalent carboxylic acids such as monomethyl maleate, monoethyl maleate, and monoisobutyl maleate. Among them, maleic anhydride is preferred.

The grafting amount of the unsaturated carboxylic acid and the anhydride thereof is preferably in a range of 0.1 to 5 wt%, and particularly preferably in a range of 0.1 to 2 wt%. If the grafting amount exceeds the aforementioned range, the adhesion strength tends to decrease.

The adhesive resin preferably has a melt flow rate (ASTM D1238 at 190°C) in a range of 0.4 to 30 g/10 min., and particularly in a range of 1 to 20 g/10 min.

When an amorphous polyester resin is used for the intermediate layer, a known resin capable of adhering to both of the polyester resin and the polyolefin resin can be used as the adhesive resin. Examples of such known resin include an ethylene-α-olefin copolymer resin and an acid-modified resin thereof, an acid-modified resin of polyolefin resin, and a glycidyl group-containing resin. These can be used singly or as a blended resin of two or more thereof.

The copolymerization of ethylene with α-olefin is not limited, but may be any of random, block, and graft copolymerization. The acid modification is typically performed by grafting unsaturated carboxylic acids such as acrylic acid, methacrylic acid, maleic acid, fumaric acid, itaconic acid, and crotonic acid, or anhydrides thereof.

It is also possible to blend a tackifier to a polyolefin resin such as a low-density polyethylene, thereby preparing an adhesive resin. Examples of the tackifier include a rosin resin, a terpene resin, and a petroleum resin. These can be blended singly or as a combination of two or more in the adhesive resin layer 2.

### [Layer constitution]

The layer constitution of the inner bag of the double-structure aerosol container of the present invention is not limited in particular as long as it has inner and outer layers formed of a polyolefin resin and an intermediate layer formed of the aforementioned cyclic olefin copolymer or the amorphous polyester resin. The present invention does not exclude the possibility of further providing any conventionally known layers such as a regrind resin-containing intermediate layer that contains a regrind resin like a scrap resin and a layer formed of nylon or the like excellent in impact resistance, though those are not essential components.

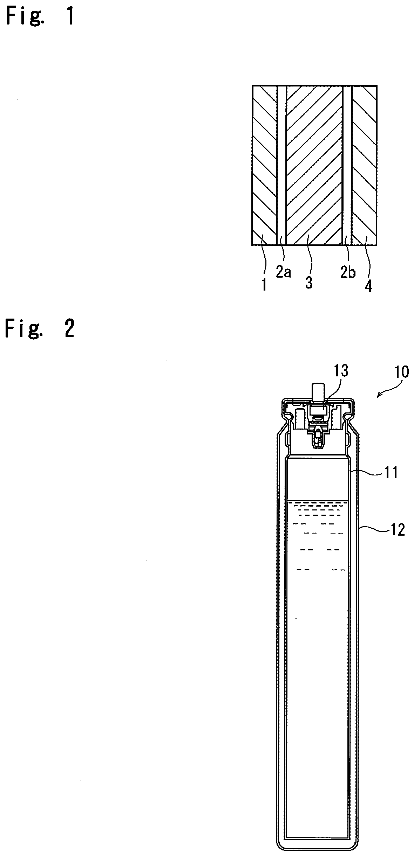

Preferably, the following layer constitution as shown in Fig. 1 can be used: linear low-density polyethylene inner layer 1 / adhesive layer 2a / intermediate layer 3 comprising cyclic olefin copolymer or amorphous polyester resin / adhesive layer 2b / linear low-density polyethylene outer layer 4.

In the present invention, the thickness of each layer is not limited in particular. Preferably, the thickness of each of the inner layer and the outer layers is in a range of 60 to 350 µm. The thickness of the intermediate layer is preferably in a range of 10 to 70 µm when a layer formed of a cyclic olefin copolymer is used as the intermediate layer. When a layer formed of an amorphous polyester resin is used as the intermediate layer, the thickness of the intermediate layer is in a range of 10 to 70 µm. In such cases, the thickness at the body portion of the inner bag (total thickness) is preferably in a range of 300 to 700 µm.

The inner bag used for the double-structure aerosol container of the present invention is preferably shaped by a melt shaping known per se, using a parison having the aforementioned laminated structure. For instance, the inner bag can be produced by so-called direct-blow forming including steps of melt-extruding a parison having a multilayer structure, placing the parison into split molds, and blowing a fluid thereinto to expand the parison. In producing a multilayer extrusion formed body, co-extrusion process known per se can be used. For instance, the process can be conducted by using the numbers of extruders to correspond to the type of the resins and using a multilayer multiple die to perform extrusion forming.

Alternatively, laminated sheets having the aforementioned laminated structure may be shaped by an ordinary process, stacked on the top of another, and heat-sealed to shape a pouch.

### (Double-structure aerosol container)

In the present invention, the aforementioned inner bag can be used for known double-structure aerosol container which is conventionally used for a two-agent type hair cosmetic.

An example of the double-structure aerosol container is a double-structure aerosol container which is capable of simultaneously ejecting the two agents by a propellant including an inactive gas such as nitrogen gas and which has: two inner bags each filled with a first agent and a second agent of the two-agent type hair cosmetic and housed in an outer vessel like a metal can; and a lid which has an ejection mechanism and with which the double-structure aerosol container is crimped. An alternative example is a duplex double-structure aerosol container formed by connecting two double-structure aerosol containers which houses each of an inner bag filled with the first agent and another inner bag filled with the second agent. In the present invention, the duplex double-structure aerosol container is particularly preferred.

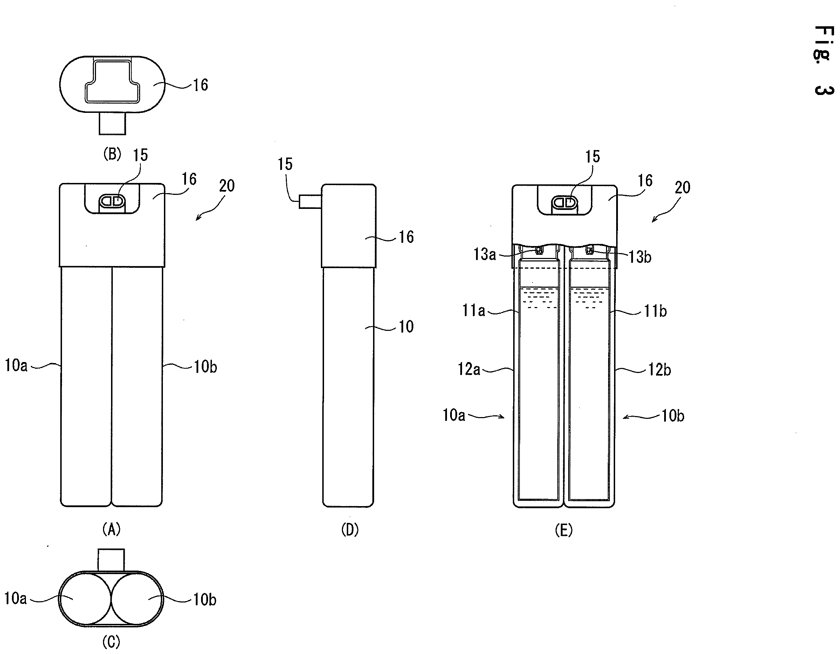

Figs. 2 and 3 explain a duplex double-structure aerosol container. Fig. 2 shows a double-structure aerosol container filled with each of the first agent and the second agent. Fig. 3 shows a duplex double-structure aerosol container formed by connecting two of the double-structure aerosol containers as shown in Fig. 2.

As shown in Fig. 2, a double-structure aerosol container indicated as 10 as a whole has a double structure formed by housing an inner bag 11 in an outer vessel 12 such as a metal can. After attaching a valve 13 at an opening of the inner bag 11, the inner bag 11 is inserted into the outer vessel 12, and subsequently a gap between the inner bag 11 and the outer vessel 12 is filled with a propellant including an inactive gas such as nitrogen gas, and the openings of the outer vessel 12 and the inner bag 11 are crimped with the valve 13.

Further as shown in Fig. 3, a duplex double-structure aerosol container 20 has a pair of double-structure aerosol containers 10a and 10b having the constitution as shown in Fig. 2, which are integrated with an outer cap 16 having an ejection port 15 which is connected to the valves 13a, 13b of these double-structure aerosol containers 10a and 10b and which is capable of simultaneously ejecting the materials filled in the inner bags 11a, 11b.

In this duplex double-structure aerosol container, the first agent and the second agent are contained independently in separate outer vessels, thereby facilitating the handling such as filling with the content. Furthermore, the two agents are simultaneously ejected and mixed at a constant ratio. As a result, a user can use the container easily by one-touch operation.

Since the inner bag of the present invention has an excellent barrier property against organic acids and hydrogen peroxide, as mentioned above, the inner bag can be used particularly preferably for the second agent containing polyoxyethylene alkyl ether, and can also be filled with the first agent containing an alkaline component.

Further, in the aforementioned duplex double-structure aerosol container, since the inner bags filled with the first agent and the second agent are housed in separate metal outer vessels, the inner bag to be filled with the first agent can comprise a single layer of a polyolefin resin. This may provide another advantage from an economic viewpoint.

For the outer vessel of the double-structure aerosol container, conventionally used metallic containers formed from a light metal plate comprising aluminum and the like or any type of surface-treated steel sheet can be used. In the present invention, an aluminum container is preferably used since corrosion can be prevented or controlled even when an outer vessel is formed from the light metal plate comprising aluminum and the like, while corrosion of the light metal particularly due to organic acids is serious.

The metal container is preferably a seamless can such as a drawn and ironed can, though any conventionally known metal containers such as weld cans can be used without any particular limitations.

The inner surface of the outer vessel is preferably covered with a coating film of conventionally known coatings such as epoxy-phenol coating and polyester coating, and is also preferably coated with a polyester resin.

The double-structure aerosol container of the present invention can contain a conventionally known two-agent type hair cosmetic without particular limitations, where the two-agent type hair cosmetic is composed of a first agent containing an alkaline component as a main component and a second agent containing hydrogen peroxide as a main component. Examples of the two-agent type hair cosmetic include a two-agent type hair dye and a two-agent type hair bleaching agent, though the present invention is not limited to these examples.

An alkaline component-containing first agent of the two-agent type hair cosmetic contains oxidation dye intermediates such as ammonia, phenylenediamines and aminophenols, and direct dyes such as 4-nitro-o-phenylenediamine and 1,4-diaminoanthraquinone.

The second agent including an oxidizing acidic component can be a conventionally known second agent containing hydrogen peroxide as an oxidizing agent, metal sequestering agents such as citric acid and EDTA, and a pH regulator. The conventionally known second agent may contain at least one of acetic acid, formic acid, and oxalic acid. In particular, the present invention has the advantage of excellent corrosion resistance to polyoxyethylene alkyl ether that may generate organic acids, particularly oxalic acid, and thus preferably be used for containing the second agent that contains the polyoxyethylene alkyl ether.

### EXAMPLES:

### (Examples 1 and 2)

A multilayer parison prepared by extrusion forming was melt-blow shaped in a mold, thereby producing a multilayered bottle having a layer constitution of: linear low-density polyethylene layer / adhesive layer / intermediate layer / adhesive layer / linear low-density polyethylene layer in this order from the innermost side. The nozzle portion of this multilayered bottle was cut to provide an inner bag for an aerosol container. The inner bag had a capacity of 45 ml.

For this inner bag, permeation amounts of organic acid and the second agent were measured by a method mentioned below and a corrosion test was conducted by using the second agent. The results were shown in Table 1.

Details of the materials and the layer constitutions of the inner bags prepared in Examples 1 and 2 are indicated below.

### Example 1

Layer constitution: five layers of three types
(Outer side) LLDPE-1 / AD-1 / COC / AD-1 / LLDPE-1 (inner side)
(Outer side) 250 / 10 / 40 / 10 / 250 (unit: µm) (inner side)

### Example 2

Layer constitution: five layers of three types
(Outer side) LLDPE-1 / AD-2 / PETG / AD-2 / LLDPE-1 (inner side)
(Outer side) 245 / 10 / 50 / 10 / 245 (unit: µm) (inner side)
Linear low-density polyethylene (LLDPE-1): NUCG-7645 manufactured by NUC Corporation
Cyclic olefin copolymer (COC): APEL APL6509T manufactured by Mitsui Chemicals, Inc.
Adhesive resin (AD-1): Evolue SP0511 manufactured by Prime Polymer Co., Ltd.
CHDM modified polyethylene terephthalate (PETG): Easter GN001 manufactured by Eastman Chemical Company
Adhesive resin (AD-2): MODIC F573 manufactured by Mitsubishi Chemical Corporation

### (Comparative Examples 1 and 2)

Linear low-density polyethylene was extruded to obtain a parison, which was then melt-blow shaped in a mold to prepare a single layer bottle. The nozzle portion of this single layer bottle was cut to produce an inner bag for an aerosol container. The inner bag had a capacity of 45 ml, and a thickness of the inner bag at the body portion was 560 µm.

For this inner bag, permeation amounts of organic acid and the second agent were measured by a method mentioned below and a corrosion test was conducted by using the second agent. The results were shown in Table 1.

Materials of the inner bags prepared in Comparative Examples 1 and 2 are as follows.

### Comparative Example 1

Linear low-density polyethylene (LLDPE-1): NUCG-7645 manufactured by NUC Corporation

### Comparative Example 2

Linear low-density polyethylene (LLDPE-2): GS-650 manufactured by NUC Corporation

### (Organic acid permeation test)

In each of the inner bags prepared in Examples 1, 2 and Comparative Examples 1, 2, 10 g of organic acid aqueous solutions containing 500 ppm of acetic acid, 500 ppm of formic acid or 100 ppm of oxalic acid, respectively was introduced. After heat-sealing the inner bags and washing the outer surfaces thereof, the inner bags were subjected to natural drying to prepare two inner bag samples for each of Examples and

### Comparative Examples.

After introducing the two inner bag samples and 60 ml of ultrapure water in a glass bottle, the bottle was capped and stored for 6 months under an environment of 45°C. After that, the water in the glass bottle was subjected to measurement of organic acid amount by ion chromatography. The conditions for ion chromatography are as follows.

### Measurement condition

Ion chromatography equipment: ICS-2100 manufactured by Nippon Dionex K.K.
Separation column: IonPac AS11-HC
Eluent: potassium hydroxide
Eluent flow rate: 1.1 ml/min.
Detector: electrical conductivity detector
Sample introduction amount: 25 µl
Analysis time: 35 minutes

### (Oxalic acid permeation test)

In the organic acid permeation test, the permeation amount of oxalic acid was below the detection limit, and thus, an additional permeation test was conducted for oxalic acid.

After loading an opening of an inner bag prepared in each of Examples 1, 2 and Comparative Examples 1, 2 with an ejection valve, the inner bag was inserted into an aluminum DI can having a capacity of 70 ml, and a gap between the inner bag and the aluminum DI can was filled with N₂ gas at a pressure of 0.3 MPa. After that, a mouth portion was tightly sealed by crimping, and the inner bag was filled with 40 g of a second agent containing hydrogen peroxide and polyoxyethylene (POE) alkylether through the valve, thereby providing an aerosol container filled with the second agent.

This aerosol container filled with second agent was stored for six months under an environment of 45°C. After that, the side surface of the aluminum DI can was pierced through which ultrapure water was introduced at an amount of 25 mass% of the second agent content. The outer surface of the inner bag and the inner surface of the aluminum DI can were shake-washed, and oxalic acid concentration in the washing water was measured by ion chromatography. The ion chromatography was conducted under the same condition as in the organic acid permeation test.

### (Corrosion test)

The aluminum DI cans after storage of the aforementioned aerosol containers filled with the second agent for six months under an environment of 45°C was opened to count the number of corrosion points of the metal surfaces caused by corrosion on the inner surface of the aluminum DI cans, by use of a stereo microscope.

**[Table 1]**

| | Organic acid permeation test | | | Oxalic acid permeation test | Corrosion test |
|---|---|---|---|---|---|
| | Permeation amount of organic acid (ppm) | | | Permeation amount of oxalic acid from second agent (ppm) | Number of corrosion points |
| | Acetic acid | Formic acid | Oxalic acid | | |
| Ex. 1 | 0.1 | 0.2 | N.D | 0.3 | 0 |
| Ex. 2 | 0.9 | 1.9 | N.D | 0.2 | 0 |
| Comp. Ex. 1 | 4.2 | 1.8 | N.D | 1.6 | 3 |
| Comp. Ex. 2 | 3.7 | 2.2 | N.D | 1.6 | 5 |

### Industrial Applicability

In a conventional technique, corrosion of an inner surface of an outer vessel is noticeable when polyoxyethylene alkyl ether is contained in the second agent for a two-agent type cosmetic. The double-structure aerosol container of the present invention can effectively prevent such corrosion, and thus, it can preferably be used for a two-agent type cosmetic which has a second agent containing polyoxyethylene alkyl ether together with hydrogen peroxide.

### Description of Reference Numerals:

1: polyolefin resin inner layer
2: adhesive layer
3: intermediate layer formed of cyclic olefin copolymer or amorphous polyester resin
4: polyolefin resin outer layer
10: double-structure aerosol container
11: inner bag
12: outer vessel
13: valve
15: ejection port
16: outer cap
20: duplex double-structure aerosol container

## Claims

1. A double-structure aerosol container for a two-agent type hair cosmetic, having an outer vessel and an inner bag to be housed in the outer vessel, wherein
the inner bag includes an inner layer and an outer layer both of which are formed of a polyolefin resin and an intermediate layer formed of a cyclic olefin copolymer or an amorphous polyester resin, and
the two-agent type hair cosmetic includes a second agent that contains hydrogen peroxide as a major ingredient and that contains at least one of an organic acid and an organic acid compound.

2. The double-structure aerosol container according to claim 1, wherein the amorphous polyester resin is glycol-modified polyethylene terephthalate.

3. The double-structure aerosol container according to claim 1 or 2, wherein the second agent contains polyoxyethylene alkyl ether.

4. The double-structure aerosol container according any one of claims 1 to 3, wherein the organic acid and an organic acid in the organic acid compound are at least one of acetic acid, formic acid and oxalic acid.

5. The double-structure aerosol container according any one of claims 1 to 4, wherein the inner bag has a thickness in a range of 300 to 700 µm.

6. The double-structure aerosol container according any one of claims 1 to 5, wherein the outer vessel is made from an aluminum seamless can.

7. The double-structure aerosol container according any one of claims 1 to 6, wherein a concentration of oxalic acid in a gap between the outer vessel and the inner bag filled with the second agent is not more than 0.5 ppm after storage for six months at 45°C.
